# EUROPÄISCHE PATENTANMELDUNG

(11) **EP 4 279 035 A2**
(43) Veröffentlichungstag der Anmeldung: **22.11.2023**
(21) Anmeldenummer: 23202374.7
(22) Anmeldetag: 16.11.2018
(51) Int. Cl.: A61F 2/50

(54) **COMPUTERIMPLEMENTIERTES VERFAHREN UND SYSTEM ZUR HERSTELLUNG EINER ORTHOPÄDISCHEN VERSORGUNG**

(30) Priorität: 27.12.2017 DE 102017131323
(62) Teilanmeldung aus: 18804596.7
(71) Anmelder: Mecuris GmbH, 80337 München (DE)
(72) Erfinder: Opitz, Manuel, 80469 München (DE); Gundlack, Felix, 91074 Herzogenaurach (DE); Schnaubelt, Max, 80686 München (DE); Rieth, Clemens, 71032 Böblingen (DE)
(74) Vertreter: Meissner Bolte Partnerschaft mbB

(57) **Zusammenfassung**

Die Erfindung betrifft ein Computerimplementiertes Verfahren zur Herstellung einer orthopädischen Versorgung, umfassend die Schritte:
a. Empfangen mindestens eines Datensatzes mit Patientendaten (ScanData);
b. Verarbeitung der Patientendaten (ScanData) zur Erstellung eines Patientenmodells;
c. Nutzung des Patientenmodells zur Bestimmung von Patientenparametern (P1, P2);
d. Generierung einer virtuellen Darstellung der orthopädischen Versorgung unter Verwendung der Patientenparameter (P1, P2) und von Versorgungsparametern (V1, V2);
e. Empfangen mindestens einer Eingabe von mindestens einem Benutzer;
f. Modifikation mindestens eines der Patientenparameter (P1, P2) und/oder Versorgungsparameter (V1, V2) basierend auf der Eingabe;
g. Physische Erstellung der orthopädischen Versorgung.

## Beschreibung

Die Erfindung betrifft ein computerimplementiertes Verfahren zur Herstellung einer orthopädischen Versorgung sowie ein entsprechendes System zur Herstellung einer orthopädischen Versorgung.

Orthopädische Versorgungen umfassend Orthesen (korrektiv und präventiv) sowie Prothesen (Exo- und Endo-Prothesen). Prothesen sind heutzutage meist aus modularen Systemen aufgebaut. Dabei werden verschiedene Grundelemente (beispielsweise Prothesenschaft, Prothesenfuß/-hand und Prothesenohr) aufgebaut, welche über standardisierte Adapter zusammengeschraubt sind. Um den technischen Aufbau wird in der Regel eine kosmetische Versorgung erstellt, welche so gut wie möglich das natürliche Bein oder den Arm imitiert. Insofern besteht ein Bedürfnis darin, das Erscheinungsbild der Versorgung so gut wie möglich an die natürlichen Gegebenheiten anzupassen. Dennoch lässt sich der Unterschied zu einer natürlichen Extremität häufig nicht verbergen. Insofern gibt es Bestrebungen, solche Versorgungen an individuelle Bedürfnisse bzw. Präferenzen des Patienten bzw. Träger anzupassen.

Ein Beispiel einer entsprechenden orthopädischen Versorgung ist aus der DE 20 2017 000 442 U1 bekannt.

Auch Endo-Prothesen werden heute individuell an den Patienten von einem oder mehreren medizinischen Experten angepasst. Hierbei ist die ästhetische Anpassung naturgemäß weit weniger wichtig als die individualisierte Form und Funktion. Eine bemusterte Oberfläche ist hier eher funktional, da so z.B. das Einwachsverhalten verbessert werden kann.

Es ist bekannt, entsprechende orthopädische Versorgungen, insbesondere Orthesen computergestützt herzustellen. Insbesondere die abschließende Fertigung der Orthese wird in der Zwischenzeit zunehmend von computergestützten Verfahren begleitet (vgl. US 2014/0180185 A1). Es ist daher oft möglich, sehr individuelle Orthesen herzustellen, wobei die die Versorgung beschreibenden Versorgungsparameter persönliche Präferenzen sowie funktionelle Angaben enthalten. Die Generierung des Modells der orthopädischen Versorgung berücksichtigt aber im Stand der Technik häufig nur sehr wenige Informationen, die von dem Patienten und/oder dem die Fertigung der Orthese betreuenden Fachpersonal, z. B. dem Orthopädietechniker, Orthopäden oder Arzt, bereitgestellt werden. Es besteht daher ein Bedarf, entsprechende Orthesen weiter zu individualisieren und die Interaktion zwischen den beteiligten Personen und Systemen zu verbessern.

Selbiges gilt auch für präventive Orthesen (Präventhesen, Protektoren). Diese können aus medizinischen Gründen aber auch zum Schutz vor Verletzungen z.B. auch bei Kontakt-, Extrem- oder Motorsportarten getragen werden. Insb. eine ästhetische Anpassung (Individualisierung) kann bei nicht zwingend medizinisch notwendigen Versorgungen ein wichtiges Kriterium für die (Kauf-)Entscheidung sein. Diese präventiven Orthesen sollen den Patienten oder Träger in seiner Bewegung gezielt vor Verletzungen schützen oder vor externen Kräften oder Überbelastungen schützen. Dies kann beispielsweise eine individualisierte präventive Orthese nach einer Knieverletzung sein, die eine dosierte Belastung des Knies bei der Rehabilitation oder beim Sport erlaubt. Denkbar ist auch eine individualisierte Zervikalorthese als Protektor für Motorradfahrer oder Rennsportpiloten, welche diese vor Verletzungen bei Stürzen oder Unfällen schützt. Weitere Beispiele sind individualisierte Protektoren für Kontaktsportler wie Fußballspieler, Eishockeyspieler, American Football, Lacrosse u.v.m. sowohl im Amateur- aber insb. im Profi-Bereich.

Ausgehend von diesem Stand der Technik ist es Aufgabe der vorliegenden Erfindung, ein verbessertes Verfahren zur Herstellung einer orthopädischen Versorgung anzugeben. Insbesondere sollen Möglichkeiten geschaffen werden, um hochgradig individualisierte Versorgungen, vorzugsweise in einem iterativen Prozess, herzustellen. Weiterhin sollen die Funktion und die Akzeptanz entsprechender Versorgungen durch die Bereitstellung eines entsprechenden Verfahrens verbessert werden.

Die vorliegende Erfindung löst dieses Problem durch die Bereitstellung des computerimplementierten Verfahrens gemäß dem Anspruch 1.

Insbesondere wird die Aufgabe durch ein computerimplementiertes Verfahren zur Herstellung einer orthopädischen Versorgung gelöst, das die nachfolgenden Schritte umfasst:
a) Empfangen mindestens eines Datensatzes mit Patientendaten;
b) Verarbeitung der Patientendaten zur Erstellung eines Patientenmodells;
c) Nutzung des Patientenmodells zur Bestimmung von Patientenparametern;
d) Generierung einer (virtuellen) Darstellung der orthopädischen Versorgung unter Verwendung der Patientenparameter und von Versorgungsparametern;
e) Empfangen mindestens einer Eingabe von mindestens einem Benutzer;
f) Modifikation mindestens eines der Patientenparameter und/oder Versorgungsparameter basierend auf der Eingabe;
g) Physische Erstellung der orthopädischen Versorgung unter Verwendung der Versorgungsparameter, Patientenparameter und/oder eines basierend auf den Patientenparametern generieten Modells der orthopädischen Versorgung.

Ein Gedanke der vorliegenden Erfindung besteht also darin, Rohdaten, wie sie beispielsweise ein Tomograph oder ein (optischer) Scanner liefert, zu nutzen, um ein Patientenmodell zu generieren.

Es kann also ein optisches Scanverfahren, z.B. unter Verwendung eines Laserscanners, eines laser-gestützten Scanners, oder eines stereoskopischen optischen Scanners mit Streifenlicht zur Gewinnung der Rohdaten durchgeführt werden. In einer Ausführungsform werden hierfür Kameras, insbesondere stereoskopische optische Kameras und/oder Kameras mit nur einem Linsensystem für digitale 3D-Rekonstruktion aus Bilderreihen eingesetzt. Nicht-optische Scanverfahren können ebenso zur Gewinnung der Rohdaten verwendet werden. Hierfür kann eine Computertomographie oder eine Magnetresonanztomographie durchgeführt werden.

Zusätzlich oder alternativ können die notwendigen Patientendaten auch manuell z.B. mittels eines Maßbands direkt am Patienten oder durch Vermessung eines Patientenabdrucks, z.B. eines Gipsnegativs, gewonnen werden. Auch taktile Vermessungsmöglichkeiten wie Messschieber, Lehren oder automatisierte taktile Messmaschinen sind denkbar.

Dieses Patientenmodell kann dann verwendet werden, um Patientenparameter abzuleiten, die letztendlich für die optimale Funktion der Versorgung notwendig sind.

Ein weiterer Gedanke der Erfindung besteht darin, Eingabe eines Benutzers, sei es eine Eingabe eines Orthopädietechnikers oder des Patienten, bei der Herstellung der Versorgung zu berücksichtigen. Entsprechende Eingaben können zur Verbesserung der Funktion sowie der Akzeptanz der Versorgung stark beitragen.

Das physische Erstellen der orthopädischen Versorgung kann ein Steuern mindestens einer Herstellungsmaschine, insbesondere eines 3D-Druckers umfassen. Allgemein sind auftragende Verfahren in diesem Bereich der Technik zu bevorzugen und führen zu Versorgungen, die bei geringem Gewicht äußerst stabil sind. Erfindungsgemäß können aber auch abtragende Verfahren, z.B. unter Verwendung einer CNC-Fräse eingesetzt werden. Auch kombinierte Verfahren bei denen unterschiedliche Drucker und/oder Herstellungsmaschinen verwendet werden sind denkbar.

Ein weiterer Gedanke der Erfindung besteht darin, den Herstellungsprozess zu optimieren. Dies setzt voraus, dass sowohl der die Herstellung betreuende Orthopädietechniker sowie der betroffene Patient möglichst intensiv in den Herstellungsprozess eingebunden ist. Daher schlägt die vorliegende Erfindung eine Visualisierung des Patientenmodells und/oder der Versorgung bzw. eines Modells der Versorgung vor.

Erfindungsgemäß können aber auch zusätzlich oder alternativ einzelne Patienten- und/oder Versorgungsparameter visualisiert werden. Hierfür können (einfache) Graphiken ggf. mit Beschriftungen verwendet werden. Beispielsweise können graphisch illustrierte Messblätter, wie sie in diesem Gebiet der Technik bekannt sind, angezeigt werden.

Die Visualisierung kann interaktiv oder statisch sein. Eine Visualisierung kann mittels einem 2D- oder einem 3D-Modell erfolgen. In einer Ausführungsform erfolgt ein Ausdrucken des Patienten- und/oder Versorgungsmodells, z.B. in 2D auf Papier oder 3D auf einem 3D-Drucker.

In einer Ausführungsform soll diese Visualisierung zu einem möglichst frühen Zeitpunkt stattfinden. In einer Ausführungsform wird eine virtuelle Darstellung des Patientenmodells generiert, wobei die orthopädische Versorgung zusammen mit dem Patientenmodell dargestellt wird. Eine entsprechende Darstellung kann unter Verwendung eines Webservers erfolgen. So lassen sich Bilder oder gar 3D-Daten mittels eines Webbrowsers visualisieren. In einer anderen Ausführungsform kann ein lokales Programm auf dem Computer des Nutzers (Orthopädietechniker oder Patienten) installiert sein, um eine geeignete Darstellung des Patientenmodells und/oder der Versorgung bereitzustellen.

In einer Ausführungsform kann die Visualisierung sowohl dem medizinischen Benutzer und/oder auch dem Patienten angezeigt werden. Für eine gesteigerte Akzeptanz der orthopädischen Versorgung ist es insbesondere hilfreich, wenn der Benutzer dem Patienten die Visualisierung der orthopädischen Versorgung in 3D z.B. in einem allgemein zugänglichen System wie z.B. einem Webbrowser oder in 2D z.B. in einem allgemein lesbaren Format wie z.B. einer PDF-Datei oder einem JPG-Bild zur Verfügung stellt.

Das Programm oder der Webbrowser können auch dafür genutzt werden, um Eingaben des oder der Benutzer(s) zu erfassen. In einer Ausführungsform wird die Darstellung der virtuellen Versorgung gegenüber dem Patientenmodell ausgerichtet. Dieser Ausrichtungsprozess kann automatisiert oder teilautomatisiert erfolgen. Der Nutzer des Verfahrens kann diese Darstellung verwenden, um notwendigen Anpassungen an dem Patientenmodell oder den Patientenparametern abzuleiten. Entsprechende Anpassungen können unmittelbar am Patientenmodell oder mittelbar an den Patientenparametern vorgenommen werden. Weiterhin können seitens des Benutzers Versorgungsparameter angepasst werden, wobei der Benutzer durch die virtuelle Darstellung in seiner Auswahl unterstützt wird.

Die Patientenparameter können Parameter umfassen, die einen Halsumfang, ein Gewicht des Patienten, einen oder mehrere Winkel, z.B. einen Fußwinkel, eine Schulterbreite, aber auch eine Position eines Adapters, angeben. Ebenso können die Versorgungsparameter mindestens einen Konstruktionsparameter und/oder mindestens Funktionsparameter und/oder mindestens ein Designparameter, z. B. Farbe der orthopädischen Versorgung, verwendetes Muster, umfassen. Der beschriebene Empfang mindestens einer Eingabe von mindestens einem Benutzer kann ein Empfangen mindestens einer ersten Eingabe von einem ersten Benutzer, beispielsweise von einem Orthopädietechniker, umfassen, wobei in einer Ausführungsform das Patientenmodell und/oder mindestens ein Patientenparameter basierend auf der Eingabe des ersten Benutzers erfolgt. Das erfindungsgemäße Verfahren ermöglicht also eine Interaktion mit einem ersten Benutzer, insbesondere einem Orthopädietechniker.

Zusätzlich oder alternativ kann das Empfangen ein Empfangen mindestens einer zweiten Eingabe von mindestens einem zweiten Benutzer, beispielsweise einem Patienten, umfassen, wobei vorzugsweise ein Modifizieren mindestens eines Versorgungsparameters basierend auf der Eingabe des zweiten Benutzers erfolgt. Insbesondere in der Konstellation, in der der erste und der zweite Benutzer Eingaben tätigen, kann das erfindungsgemäße Verfahren die Synchronisation dieser Eingaben ermöglichen, so dass alle Änderungsvorschläge berücksichtigt werden.

In einer Ausführungsform implementiert das Verfahren eine Authentifizierung des ersten und/oder zweiten Benutzers, so dass keine unberechtigte oder ungewollte Veränderung der relevanten Parameter erfolgen kann. Vorzugsweise wird eine Berechtigungsdatenbank implementiert, die beispielsweise dem ersten Benutzer eine Vielzahl von Versorgungen zuordnet, die dieser bearbeiten darf. Beispielsweise kann diese Berechtigung darauf beruhen, dass der erste Benutzer die Versorgungen ursprünglich beauftragt hat. Zusätzlich oder alternativ kann die Berechtigungsdatenbank angeben, welche Parameter, insbesondere welche Versorgungsparameter, durch den zweiten Benutzer veränderbar sind. Soweit es sich bei dem zweiten Benutzer um einen Patienten handelt, können hierdurch Einschränkungen vorgenommen werden, die den zweiten Benutzer davon abhalten, funktionell relevante Parameter zu verändern. Stattdessen kann es dem zweiten Benutzer ausschließlich ermöglicht werden, Versorgungsparameter, wie beispielsweise ein Erscheinungsbild der Versorgung eingeben, zu verändern.

In einer Ausführungsform kann die Berechtigungsdatenbank auch dafür zum Einsatz kommen, bestimmten Nutzern des gleichen Typs, z.B. eines Orthopädietechnikers oder Arztes, bestimmte Berechtigungslevel zuzuweisen. So könnte z.B. ein Orthopädietechniker der bereits mehrere orthopädische Versorgungen nach dem beschriebenen Verfahren durchgeführt hat oder im System als Experte markiert ist, Zugriff auf mehre Parameter hat (Expertenmodus).

In einer Ausführungsform umfassen die Patientendaten zusätzlich Kontaktdaten eines zweiten Benutzers bzw. des Patienten. Das Verfahren kann ein elektrisches Übermitteln einer Nachricht an den Patienten umfassen, die diesen auffordert, eine oder die bereits beschriebenen Eingaben zu tätigen. In einer Ausführungsform enthält die Nachricht eine URL, die es dem Benutzer ermöglicht, eine entsprechende Eingabemaske aufzurufen. Alternativ und zusätzlich können eine Benutzerkennung und/oder ein Passwort enthalten sein. Zusätzlich oder alternativ können die Patientendaten genutzt werden, um den Patienten zu authentifizieren. Vorzugsweise kann ein Patient nur dann Eingaben tätigen, wenn er sich erfolgreich authentifizieren konnte.

Die eingangs genannte Aufgabe wird des Weiteren durch einen computerlesbaren Speicher mit Instruktionen zur Implementierung eines der bereits beschriebenen Verfahren gelöst, wenn die Instruktionen auf mindestens einer Recheneinheit ausgeführt werden.

Es ergeben sich ähnliche Vorteile, wie diese bereits in Verbindung mit dem Verfahren beschrieben wurden.

Weiterhin wird die Aufgabe durch ein System zur Herstellung einer orthopädischen Versorgung gelöst, das vorzugsweise zumindest einige der Schritte, die in Verbindung mit dem Verfahren beschrieben wurden implementiert.

In einer Ausführungsform handelt es sich bei dem System um ein System mit:
einem Entwurfsserver, der umfasst:
   - mindestens eine digitale Schnittstelle zum Empfangen eines Datensatzes mit Patientendaten;
   - mindestens eine Datenbank mit Trainingsdaten zur Erstellung eines Patientenmodells basierend auf den Patientendaten und den Trainingsdaten;
   - mindestens eine Recheneinheit zur Generierung von 3D-Daten der orthopädischen Versorgung; und
   - einer Visualisierungseinrichtung, insbesondere einem Webserver, der dazu ausgebildet ist, die 3D-Daten in Form einer virtuellen Darstellung der Versorgung darzustellen und mindestens eine Eingabe mindestens eines Benutzers zu empfangen;
wobei die Recheneinheit, die Eingabe dazu verwendet,
   a) Versorgungsparameter und/oder Patientenparameter zu modifizieren und
   b) basierend auf den Versorgungsparametern und den Patientenparametern Herstellungsdaten zur physischen Erstellung der orthopädischen Versorgung zu erzeugen.

Auch bei dem System ergeben sich ähnliche oder identische Vorteile, wie diese bereits in Verbindung mit dem Verfahren beschrieben wurden.

Nachfolgend wird die Erfindung mittels mehrerer Ausführungsbeispiele beschrieben, die anhand von Abbildungen näher erläutert werden.

Hierbei zeigen:
- Fig. 1: einzelne Komponenten eines Systems zur Herstellung einer orthopädischen Versorgung;
- Fig. 2: Elemente des Herstellungsservers aus Fig. 1;
- Fig. 3: einzelne Verfahrensschritte zur Herstellung einer orthopädischen Versorgung;
- Fig. 4: Beispiel einer erfindungsgemäß gewonnenen Fußprothese; und
- Fig. 5: erfindungsgemäße Visualisierung der Fußprothese aus Fig. 4.

In der nachfolgenden Beschreibung werden für gleiche und gleichwirkende Teile dieselben Bezugsziffern verwendet.

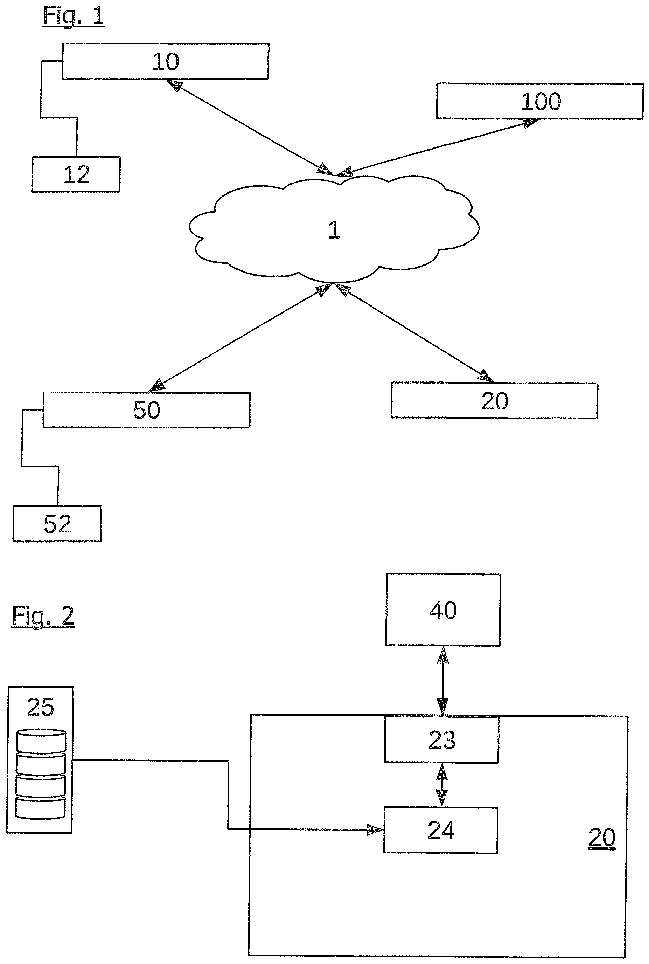

Fig. 1 zeigt einige der Komponenten, die im Zuge eines Ausführungsbeispiels des erfindungsgemäßen Herstellungsverfahrens miteinander kommunizieren. Hierbei handelt es sich um einen CPO-Computer 10, einen Patientencomputer 100, einen Herstellungsserver 50 sowie einen Entwurfsserver 20. All diese Komponenten sind kommunikativ über ein Netzwerk, in dem beschriebenen Ausführungsbeispiel über das Internet 1 miteinander verbunden.

Der CPO-Computer 10 umfasst einen optischen Scanner 12, zur Erfassung der Oberflächenstruktur eines Patienten. Hierdurch ergeben sich Rohdaten (ScanData).

Der Herstellungsserver 50 verfügt im beschriebenen Ausführungsbeispiel über einen 3D-Drucker 52 und kann somit eine beliebige Orthese herstellen, soweit ihm die hierfür notwendigen Daten von dem Entwurfsserver 20 bereitgestellt werden.

Die einzelnen Bestandteile des Entwurfsservers 20 werden näher anhand der Fig. 2 erläutert. Der Entwurfsserver 20 verfügt über eine Recheneinheit 24, die das nachfolgend beschriebene Verfahren zumindest teilweise implementiert. Des Weiteren ist eine Entwurfsschnittstelle 23 zur Kommunikation mit dem bereits beschriebenen CPO-Computer 10 und dem Patientencomputer 100 vorgesehen. In einem bevorzugten Ausführungsbeispiel erfolgt diese Kommunikation über einen Webserver 40, so dass die Computer 10, 100 keine eigene Software zur Kommunikation mit dem Entwurfsserver 20 benötigen. So kann auf die von dem Entwurfsserver 20 bereitgestellten Dienste mittels eines Webbrowsers zugegriffen werden.

Des Weiteren ist eine Datenbank 25 vorgesehen. Diese Datenbank 25 kann notwendige Modelldaten liefern, um seitens des Entwurfsservers 20 Modelle der Orthese herzustellen. Weiterhin können auf der Datenbank 25 Templates oder Parameter gespeichert sein, die es ermöglichen, ein individuelles Patientenmodell herzustellen. Die Datenbank 25 kann weiterhin Authentifizierungsinformationen enthalten, um Zugriffe auf den Entwurfsserver 20, insbesondere auf die von diesem angebotenen Dienste, zu verwalten.

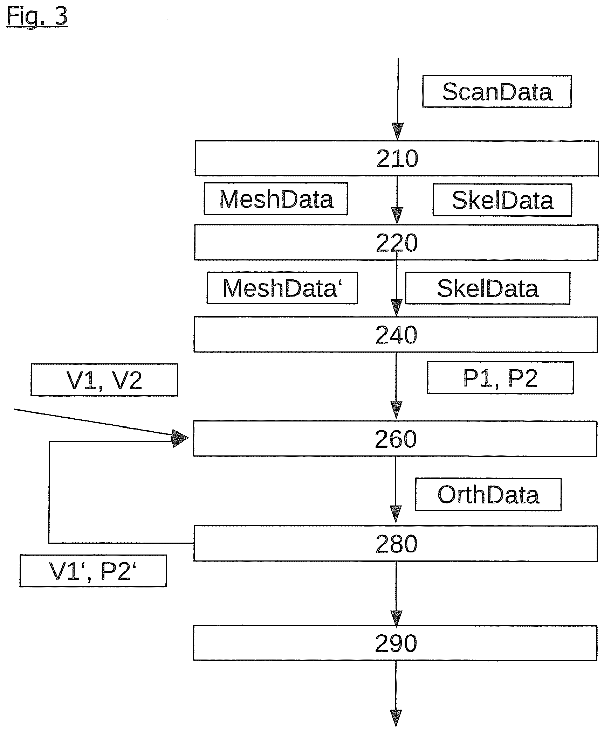

Wie anhand der Fig. 3 ersichtlich, empfängt der Entwurfsserver 20 die vom Orthopädietechniker mittels eines CPO-Computers 10 erfassten Rohdaten ScanData. Diese Rohdaten ScanData können beispielsweise im DICOM-Format bereitgestellt werden. In einem Vorverarbeitungsschritt 210 werden aus den Rohdaten ein Oberflächennetz des Patienten MeshData sowie Knochendaten SkelData gewonnen. Das Oberflächennetz MeshData - Mesh - kann als Dreiecksnetz, beispielsweise im STL Format erzeugt werden. Zur Gewinnung des Oberflächennetzes MeshData werden Oberflächenpunkte aus den Rohdaten ScanData extrahiert und die gewonnene Punktewolke in ein entsprechendes Netz eingebettet.

Auch bei den Knochendaten SkelData ist eine Modellierung als Dreiecksnetz erfindungsgemäß möglich. Bevorzugt werden aber Gelenke und Gelenkverbindungen, z.B. mittels Vektoren, modelliert und in einer geeigneten Datenstruktur erfasst. Weiterhin können zu jedem Gelenk übliche Freiheitsgrade im Hinblick auf Rotation- und/oder Translationsbewegungen gespeichert werden.

Das Oberflächennetz MeshData sowie die Knochendaten SkelData können in einem nachfolgenden Datenvalidierungs- und Optimierungsschritt 220 optimiert und validiert werden. In einem Ausführungsbeispiel erfolgt in dem Schritt 220 ein Anzeigen der Daten, wobei automatisch oder computergestützt Korrekturen vorgenommen werden. Basierend auf den vorgenommenen Korrekturen ergibt sich ein korrigiertes Oberflächennetz MeshData'. Die Korrektur kann ein Ausrichten der Knochendaten SkelData gemäß einer vorgegebenen, ggf. standardisierten Ausrichtung, umfassen, wobei das korrigierte Oberflächennetz MeshData' entsprechend der Ausrichtung der Knochendaten SkelData verformt wird.

Das korrigierte Oberflächennetz MeshData' sowie die Knochendaten SkelData werden in einem Patientenparameter-Extraktionsschritt 240 verarbeitet. Vorzugsweise wird in diesem Schritt 240 unter Zuhilfenahme der Datenbank 25 ein Patientenmodell gewonnen. Basierend auf diesem Patientenmodell werden Patientenparameter P1, P2 abgeleitet. Diese Patientenparameter P1, P2 können im Schritt der Orthesen-Modellerzeugung 260 verwendet werden, um ein Modell der Orthese zu erzeugen. Vorzugsweise liegen bereits einige Versorgungsparameter V1, V2 vor, die Parameter der Orthese angeben. Das Orthesenmodell und ggf. auch das Patientenmodell können in einem Visualisierungsschritt 220 visualisiert werden.

In einem Ausführungsbeispiel kann die Visualisierung genutzt werden, um einige der Parameter, beispielsweise den Versorgungsparameter V1 und den Patientenparameter P2 anzupassen. Eine entsprechende Anpassung kann durch den Orthopädietechniker oder ggf. durch den Patienten erfolgen. Die Anpassung kann in einem Schritt oder in getrennten Schritten vorgenommen werden. Nach einer Veränderung der Parameter kann in einer erneuten Orthesen-Modellerzeugung 260 ein aktualisiertes Modell der Orthese, z.B. unter Verwendung des modifizierten Versorgungsparameters V1' und des modifizierten Patientenparameters P2', erzeugt werden. Es ergeben sich Orthesen-Modelldaten OrthData, die soweit sie nach einer erneuten Visualisierung 280 die Zustimmung der Benutzer finden, dem Herstellungsserver 50 zugeführt werden, um eine Orthesen-Fertigung 290 einzuleiten.

Datenvalidierungs- und Optimierungsschritt 220 umfasst in einem Ausführungsbeispiel eine Ausrichtungskorrektur, z.B. gemäß einer bestimmten standardisierten Vorgabe.

Um die Haltung eines Scans zu korrigieren bedarf es des Oberflächennetzes MeshData und der Knochendaten SkelData. Knochendaten SkelData können, wie erläutert, ein vereinfachtes Knochengerüst sein, welches im Inneren des erfassten Objekts und somit innerhalb des Oberflächennetzes MeshData liegt. In einem Ausführungsbeispiel liegen Daten vor, die die Interaktion zwischen den Knochendaten SkelData und dem Oberflächennetz MeshData modellieren.

Beispielsweise können Vektoren Abstände oder Stützstellen innerhalb des Oberflächennetzes angeben. Entsprechende Vektoren können basieren auf Templates, die in der Datenbank 25 gespeichert sind, gewonnen werden.

Eine Bewegung der Knochen für die Ausrichtungskorrektur führt zu einer Verformung des modellierten 3D Objektes und somit zu einem veränderten Oberflächennetz.

Erfindungsgemäß können die anatomischen Gegebenheiten berücksichtigt werden. In einem Ausführungsbeispiel werden die Knochendaten SkelData unter Verwendung der Templates an das gewonnene Oberflächennetz angepasst, und durch weitere Prozessschritte verbessert, um eine möglichst realistische Verformung modellieren zu können. Das sich ergebende korrigierte Oberflächennetz MashData' kann zur Extraktion von Patientenparametern genutzt werden.

Z.B. bei der Herstellung einer patientenindividuellen Knöchelorthese (Ankle-Foot-Orthosis, AFO) kann ein Unterschenkelscan betrachtet werden. Der Scan bzw. die zugehörigen Rohdaten ScanData können erfindungsgemäß im Schritt 220 in eine korrigierte Haltung gebracht werden. Dazu wird in einem ersten Schritt die Orientierung des Fußes identifiziert und in eine definierte Orientierung gebracht.

Um die Haltung beim Scan beurteilen zu können, werden Winkel des Knochenmodelles - Knochendaten SkelData - zusammen mit weiteren biomechanischen Achsen und Ebenen untersucht. Weichen diese Winkel von einem gewählten Maß ab, werden die Knochendaten SkelData angepasst - ausgerichtet, wodurch sich auch der Scan ändert (korrigiertes Oberflächennetz MeshData).

Erfindungsgemäß können beispielsweise folgende Schritte durchgeführt werden:
- Erstellen eines Patientenmodells basierend auf den Knochendaten SkelData und den Oberflächendaten MeshData;
- Finden des Fußes in dem Patientenmodell;
- Nutzung der vorhandenen Daten, um eine Auflageebene zu bestimmen;
- Modifizieren eines auf den Knochendaten SkelData basierenden Modelles bis die Auflageebene parallel zu einer virtuellen Bodenfläche ausgerichtet ist, z.B. Rotation um das Fußgelenk (erste Ausrichtungskorrektur);
- Modifizieren des Modells bis der Winkel eines Kniegelenks einen vorgegebenen Wert einnimmt (zweite Ausrichtungskorrektur);

Modifizieren der Oberflächendaten MeshData basierend auf der ersten und zweiten Ausrichtungskorrektur zum Erhalt der korrigierten Oberflächendaten MeshData'.

Die beschriebene Ausrichtungskorrektur kann eine Extraktion von Patientendaten erst ermöglichen oder das Ergebnis deutlich verbessern.

Der Patientenparameter-Extraktionsschritt 240 folgt in einem Ausführungsbeispiel dem nachfolgenden Schema.

Für die Konstruktion einer patientenindividuellen Knöchelorthese (Ankle-Foot-Orthosis, AFO) bedarf es beispielsweise verschiedener Längen- und Umfangsmaße des Fußes und Unterschenkels (Patientenparameter). Um diese aus den Rohdaten ScanData zu extrahieren kann wie folgt vorgegangen werden:
Die Oberflächendaten MeshData des Patienten werden ausgerichtet, und in ein Referenzsystem gebracht, aus dem sich schließen lässt, welcher Teil des Scans den Fuß, und welcher das Bein darstellt. In den Oberflächendaten MeshData wird dann ein vereinfachtes Fußmodell platziert.

Dieses Fußmodell, das ggf. in der Datenbank 25 gespeichert ist, ist bekannt und lässt sich anhand seiner Freiheitsgrade - bspw. Länge, Skalierung, Rotation von Subteilen, etc. - verändern.

Die Freiheitsgrade des Fußmodells werden in einem Optimierungsverfahren an Oberflächendaten MeshData angepasst bis die Korrelation von MeshDaten und Fußmodell optimal ist (möglichst geringe Abweichung). In einem Ausführungsbeispiel wird entsprechend mit dem "Significant Points" Modell (SPM), welches zur Abnahme der Maße verwendet wird, verfahren.

Das SPM besteht aus Punkten und Ebenen, zwischen denen Maße abgenommen werden. Von dem SPM werden die Maßabnahmepunkte auf die Oberflächendaten MeshData oder die korrigierten Oberflächendaten MeshData' projiziert. Dies geschieht je nach Messart unterschiedlich. Umfangsmaße benötigen eine Schnittebene, Längenmaße lediglich Punkte.

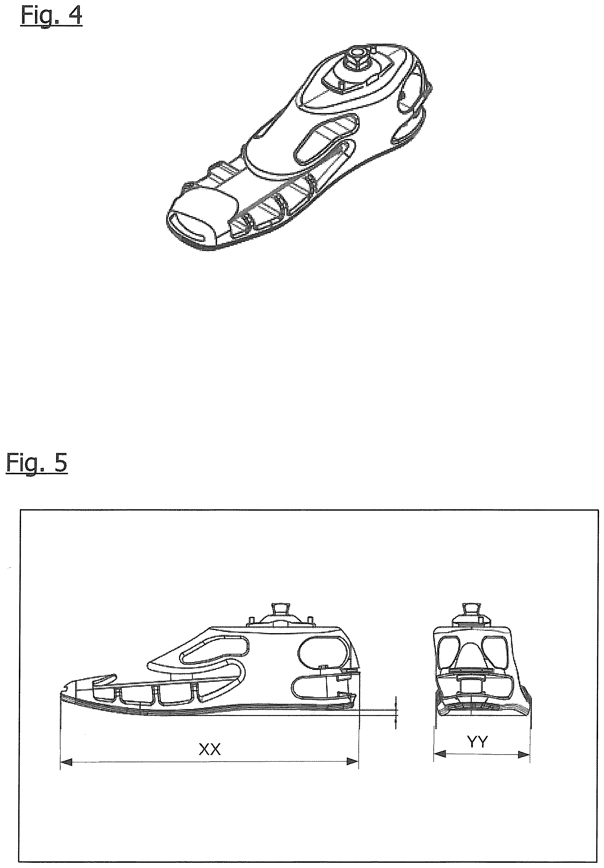

Zwischen diesen projizierten Punkten, oder entlang der Schnittebenen werden die Maße abgenommen. Diese können zum Beispiel in ein Maßtblatt eingetragen werden. In einem Ausführungsbeispiel umfasst der Visualisierungsschritt 220 das Erzeugen eines 3D-Bilds der Orthese. In einem anderen Ausführungsbeispiel wird zur Visualisierung von Patientendaten ein Maßblatt angezeigt und/oder ausgedruckt, dass ähnlich oder identisch aussieht wie dies in der Fig. 5 gezeigt ist.

Vorhergehend wurden ein Herstellungsverfahren sowie ein System zur Herstellung einer Orthese beschrieben. Mit den erfindungswesentlichen Merkmalen lässt sich auch ohne Weiteres eine Prothese, z.B. eine Fußprothese, wie diese in Fig. 4 gezeigt ist herstellen.

Auch die Herstellung von Endo-Prothesen oder präventiven Orthesen (Protektoren für Rehabilitation und Sport) nach demselben Verfahren ist denkbar.

Aspekte der vorliegenden Offenbarung umfassen das Folgende:
Aspekt 1. Computerimplementiertes Verfahren zur Herstellung einer orthopädischen Versorgung, insbesondere einer Orthese oder Prothese, umfassend die Schritte:
   a. Empfangen mindestens eines Datensatzes mit Patientendaten (ScanData);
   b. Verarbeitung der Patientendaten (ScanData) zur Erstellung eines Patientenmodells;
   c. Nutzung des Patientenmodells zur Bestimmung von Patientenparametern (P1, P2);
   d. Generierung einer virtuellen Darstellung der orthopädischen Versorgung unter Verwendung der Patientenparameter (P1, P2) und von Versorgungsparametern (V1, V2);
   e. Empfangen mindestens einer Eingabe von mindestens einem Benutzer;
   f. Modifikation mindestens eines der Patientenparameter (P1, P2) und/oder Versorgungsparameter (V1, V2) basierend auf der Eingabe;
   g. Physische Erstellung der orthopädischen Versorgung unter Verwendung der Versorgungsparameter (V1, V2), Patientenparameter (P1, P2) und/oder eines basierend auf den Patientenparametern generieten Modells der orthopädischen Versorgung (OrthData).
Aspekt 2. Verfahren nach Aspekt 1,
   **dadurch gekennzeichnet,** dass
   der Schritt f) ein Steuern mindestens einer Herstellungsmaschine (50), insbesondere mindestens eines 3D Druckers, umfasst.
Aspekt 3. Verfahren nach einem der vorhergehenden Aspekte,
   **gekennzeichnet** durch
   einen Schritt d1), in dem eine virtuelle Darstellung des Patientenmodells generiert wird, wobei in Schritt d) die orthopädische Versorgung zusammen mit dem Patientenmodell, insbesondere unter Verwendung eines Webservers, dargestellt wird.
Aspekt 4. Verfahren nach einem der vorhergehenden Aspekte,
   **gekennzeichnet** durch
   einen Schritt d2), in dem die virtuelle Darstellung des Patientenmodells (MeshData) gegenüber der virtuellen Darstellung der orthopädischen Versorgung ausgerichtet wird.
Aspekt 5. Verfahren nach einem der vorhergehenden Aspekte,
   **dadurch gekennzeichnet,** dass
   die Patientenparameter (P1, P2) Parameter umfassen, die einen Halsumfang, eine Schulterbreite, angeben und/oder
   die Versorgungsparameter (V1, V2) mindestens einen Konstruktionsparameter und/oder mindestens einen Funktionsparameter, z.B. Materialstärke, Flexibilität, und/oder mindestens einen Designparameter, z.B. Farbe der orthopädischen Versorgung, umfassen.
Aspekt 6. Verfahren nach einem der vorhergehenden Aspekte,
   **gekennzeichnet** durch:
   ein Empfangen mindestens einer ersten Eingabe von einem ersten Benutzer, insbesondere einem Arzt oder Orthopädietechniker, und ein Modifizieren des Patientenmodells und/oder mindestens eines Patientenparameters (P1, P2) basierend auf der Eingabe des ersten Benutzers.
Aspekt 7. Verfahren nach einem der vorhergehenden Aspekte,
   **gekennzeichnet** durch:
   ein Empfangen mindestens einer zweiten Eingabe von einem zweitem Benutzer, insbesondere einem Patienten, und ein Modifizieren mindestens eines Versorgungsparameters (V1, V2) basierend auf der Eingabe des zweiten Benutzers.
Aspekt 8. Verfahren nach einem der vorhergehenden Aspekte,
   **gekennzeichnet** durch:
   i. ein Authentifizieren des ersten Benutzer vor dem Empfangen der Eingabe des ersten Benutzers und/oder
   ii. ein Authentifizieren des zweiten Benutzer vor dem Empfangen der zweiten Eingabe des ersten Benutzers.
Aspekt 9. Verfahren nach einem der vorhergehenden Aspekte, insbesondere nach Aspekt 7,
   gekennzeichnet durch:
   ein Nachschlagen in einer Berechtigungsdatenbank, welche der Versorgungsparameter durch den zweiten Benutzer veränderbar sind und
   ein Anzeigen nur der Versorgungsparameter (V1, V2) zur Modifikation, die gemäß der Berechtigungsdatenbank durch den zweiten Benutzer veränderbar sind.
Aspekt 10. Verfahren nach einem der vorhergehenden Aspekte,
   **gekennzeichnet** durch
   die Patientendaten (ScanData) Kontaktdaten eines Patienten umfassen, wobei das Verfahren aufweist:
   i. elektronisches Übermitteln einer Nachricht mit einer URI an den Patienten unter Verwendung der Kontaktdaten,
   ii. Authentifizieren des Patienten unter Verwendung der Kontaktdaten und/oder Patientenparameter derart, dass der Patient als Benutzer, insbesondere als zweiter Benutzer, die mindestens eine Eingabe vornehmen kann.
Aspekt 11. Computerlesbarer Speicher mit Instruktionen zur Implementierung des Verfahrens gemäß einem der vorhergehenden Aspekte, wenn die Instruktionen auf mindestens einer Recheneinheit ausgeführt werden.
Aspekt 12. System zur Herstellung einer orthopädischen Versorgung, insbesondere einer Orthese oder Prothese, wobei vorzugsweise zumindest einige der Schritte des Verfahrens gemäß den Aspekten 1 bis 10 implementiert werden, mit:
   einem Entwurfsserver (20), der umfasst:
   - mindestens eine digitale Schnittstelle (23) zum Empfangen eines Datensatzes mit Patientendaten (ScanData);
   - mindestens eine Datenbank (25) mit Trainingsdaten zur Erstellung eines Patientenmodells basierend auf den Patientendaten (ScanData) und den Trainingsdaten;
   - mindestens eine Recheneinheit zur Generierung von 3D-Daten der orthopädischen Versorgung; und
   - einer Visualisierungseinrichtung, insbesondere einem Webserver (40), der dazu ausgebildet ist, die 3D-Daten in Form einer (virtuellen) Darstellung der Versorgung darzustellen und mindestens eine Eingabe mindestens eines Benutzers zu empfangen;
      wobei die Recheneinheit die Eingabe dazu verwendet,
      a) Versorgungsparameter (V1, V2) und/oder Patientenparameter (P1, P2) zu modifizieren und
      b) basierend auf den Versorgungsparametern (V1, V2) und den Patientenparametern (P1, P2) Herstellungsdaten zur physischen Erstellung der orthopädischen Versorgung zu erzeugen.

### Bezuaszeichenliste:

- 1: Internet
- 10: CPO-Computer
- 12: Scanner
- 20: Entwurfsserver
- 23: Entwurfsserverschnittstelle
- 24: Recheneinheit
- 25: Datenbank
- 40: Webserver
- 50: Herstellungsserver
- 52: 3D-Druck
- 100: Patienten-Computer
- 210: Vorverarbeitung (z.B. Knochendatenextraktion)
- 220: Datenvalidierung und Optimierung
- 240: Patientenparameter-Extraktion
- 260: Orthesen-Modellerzeugung
- 280: Visualisierung
- 290: Orthesen-Fertigung
- ScanData: Rohdaten
- MeshData: Oberflächennetz
- MeshData': Korrigiertes Oberflächennetz
- OrthData: 3D Orthesen-Modelldaten
- SkelData: Knochendaten
- P1, P2, P2': Patientenparameter
- V1, V2, V1': Orthesenparameter

## Patentansprüche

1. Computerimplementiertes Verfahren zur Herstellung einer orthopädischen Versorgung, umfassend die folgenden Schritte:
a) Empfangen mindestens eines Datensatzes mit Patientendaten (ScanData) betreffend die Oberflächenstruktur eines Patienten;
b) Verarbeitung der Patientendaten (ScanData) zur Erstellung eines Patientenmodells unter Verwendung von in einer Datenbank (25) gespeicherten Templates;
c) Nutzung des Patientenmodells zur Bestimmung von Patientenparametern (P1, P2);
d) Generierung einer virtuellen Darstellung der orthopädischen Versorgung unter Verwendung der Patientenparameter (P1, P2) und von Versorgungsparametern (V1, V2);
e) Empfangen mindestens einer Eingabe von mindestens einem Benutzer, umfassend eine erste Eingabe von einem ersten Benutzer und eine zweite Eingabe von einem zweiten Benutzer;
f) Modifikation mindestens eines der Patientenparameter (P1, P2) und/oder Versorgungsparameter (V1, V2) basierend auf der Eingabe, zur Erzeugung von modifizierten Patientenparametern (P2') und/oder modifizierten Versorgungsparametern (V1');
g) Physische Erstellung der orthopädischen Versorgung unter Verwendung der modifizierten Versorgungsparameter (V1') und/oder der modifizierten Patientenparameter (P2').

2. Verfahren nach Anspruch 1,
wobei die Patientenparameter (P1, P2) mindestens einen der folgenden Parameter angeben: ein Längen- oder Umfangsmaßes des Fußes oder Unterschenkels, ein Gewicht eines Patienten, einen oder mehrere Winkel, und eine Position eines Adapters.

3. Verfahren nach einem der vorhergehenden Ansprüche,
**dadurch gekennzeichnet, dass**
die Versorgungsparameter (V1, V2) mindestens einen der folgenden Parameter umfassen:
- mindestens einen Konstruktionsparameter;
- mindestens einen Funktionsparameter, z.B. Materialstärke, Flexibilität;
- mindestens einen Designparameter, z.B. Farbe der orthopädischen Versorgung.

4. Verfahren nach Anspruch 1,
**dadurch gekennzeichnet, dass**
der Schritt g) ein Steuern mindestens einer Herstellungsmaschine (50), insbesondere mindestens eines 3D Druckers, umfasst.

5. Verfahren nach einem der vorhergehenden Ansprüche,
**gekennzeichnet durch**
einen Schritt d1), in dem eine virtuelle Darstellung des Patientenmodells generiert wird, wobei in Schritt d) die orthopädische Versorgung zusammen mit dem Patientenmodell, insbesondere unter Verwendung eines Webservers, dargestellt wird.

6. Verfahren nach einem der vorhergehenden Ansprüche,
**gekennzeichnet durch**
einen Schritt d2), in dem die virtuelle Darstellung des Patientenmodells (MeshData) gegenüber der virtuellen Darstellung der orthopädischen Versorgung ausgerichtet wird.

7. Verfahren nach einem der vorhergehenden Ansprüche,
**dadurch gekennzeichnet, dass**
der erste Benutzer ein Arzt oder Orthopädietechniker ist und ein Modifizieren des Patientenmodells und/oder mindestens eines Patientenparameters (P1, P2) basierend auf der Eingabe des ersten Benutzers stattfindet.

8. Verfahren nach einem der vorhergehenden Ansprüche,
**dadurch gekennzeichnet, dass**
der zweite Benutzer ein Patienten ist und ein Modifizieren mindestens eines Versorgungsparameters (V1, V2) basierend auf der Eingabe des zweiten Benutzers stattfindet.

9. Verfahren nach einem der vorhergehenden Ansprüche,
**gekennzeichnet durch**:
i) ein Authentifizieren des ersten Benutzers vor dem Empfangen der ersten Eingabe des ersten Benutzers und/oder
ii) ein Authentifizieren des zweiten Benutzers vor dem Empfangen der zweiten Eingabe des zweiten Benutzers.

10. Verfahren nach einem der vorhergehenden Ansprüche, insbesondere nach Anspruch 9,
**gekennzeichnet durch**:
ein Nachschlagen in einer Berechtigungsdatenbank, welche der Versorgungsparameter durch den zweiten Benutzer veränderbar sind und
ein Anzeigen nur der Versorgungsparameter (V1, V2) zur Modifikation, die gemäß der Berechtigungsdatenbank durch den zweiten Benutzer veränderbar sind.

11. Verfahren nach einem der vorhergehenden Ansprüche,
**dadurch gekennzeichnet, dass**
die Patientendaten (ScanData) Kontaktdaten eines Patienten umfassen, wobei das Verfahren aufweist:
i) elektronisches Übermitteln einer Nachricht mit einer URI an den Patienten unter Verwendung der Kontaktdaten,
ii) Authentifizieren des Patienten unter Verwendung der Kontaktdaten und/oder Patientenparameter derart, dass der Patient als Benutzer, insbesondere als zweiter Benutzer, die mindestens eine Eingabe vornehmen kann.

12. Computerlesbarer Speicher mit Instruktionen zur Implementierung des Verfahrens gemäß einem der vorhergehenden Ansprüche, wenn die Instruktionen auf mindestens einer Recheneinheit ausgeführt werden.

13. System zur Herstellung einer orthopädischen Versorgung, insbesondere einer Orthese oder Prothese, wobei das Verfahren gemäß einer der Ansprüche 1 bis 11 implementiert wird, mit:
einem Entwurfsserver (20), der das Folgende umfasst:
- mindestens eine digitale Schnittstelle (23) zum Empfangen eines Datensatzes mit Patientendaten (ScanData);
- mindestens eine Datenbank (25) mit Trainingsdaten zur Erstellung eines Patientenmodells basierend auf den Patientendaten (ScanData) und den Trainingsdaten;
- mindestens eine Recheneinheit zur Generierung von 3D-Daten der orthopädischen Versorgung; und
- einer Visualisierungseinrichtung, insbesondere einem Webserver (40), der dazu ausgebildet ist, die 3D-Daten in Form einer (virtuellen) Darstellung der Versorgung darzustellen und mindestens eine Eingabe mindestens eines Benutzers zu empfangen;
wobei die Recheneinheit die Eingabe dazu verwendet,
a) Versorgungsparameter (V1, V2) und/oder Patientenparameter (P1, P2) zu modifizieren und
b) basierend auf den Versorgungsparametern (V1, V2) und den Patientenparametern (P1, P2) Herstellungsdaten zur physischen Erstellung der orthopädischen Versorgung zu erzeugen.
